Europäisches Patentamt

European Patent Office

Office européen des brevets

(11) Veröffentlichungsnummer: **0 106 374**
**B1**

(12) **EUROPÄISCHE PATENTSCHRIFT**

(45) Veröffentlichungstag der Patentschrift :
25.03.87

(51) Int. Cl.⁴ : **G 01 N 33/24**, G 01 N 1/34

(21) Anmeldenummer : 83200937.7

(22) Anmeldetag : 23.06.83

(54) Verfahren und Vorrichtung für das Absondern der Cysten von Organismen, die Krankheiten an landwirtschaftlichen Gewächsen verursachen, aus Bodenproben.

(30) Priorität : 20.07.82 NL 8202911

(43) Veröffentlichungstag der Anmeldung :
25.04.84 Patentblatt 84/17

(45) Bekanntmachung des Hinweises auf die Patenterteilung : 25.03.87 Patentblatt 87/13

(84) Benannte Vertragsstaaten :
AT BE CH DE FR GB IT LI NL SE

(56) Entgegenhaltungen :
DD-A-    44 999
DE-A- 2 551 948
US-A- 3 813 947
US-A- 4 072 275

(73) Patentinhaber : **Volkers, Aris**
**Bovenweg 41**
**Sint Pancras (NL)**

(72) Erfinder : **Volkers, Aris**
**Bovenweg 41**
**Sint Pancras (NL)**

(74) Vertreter : **Koomen, Jan, Ir.**
**Kennemerstraatweg 35**
**NL-1814 GB Alkmaar (NL)**

## Beschreibung

Die Erfindung betrifft ein Verfahren und eine Vorrichtung fuer das Absondern der Zysten von Organismen oder Aelchen, die Krankheiten an Landwirtschaftlichen Gewaechsen, wie Kartoffelmuedigkeit verursachen, aus eine Bodenprobe, wobei die Bodenprobe mit Wasser vermischt wird und die Zysten sich als leichtere Bestandteile an der Gemischoberflaeche sammeln. Zur Feststellung der Konzentration der Zysten in der Bodenprobe wird ein Teil des Gemisches auf ein Sieb geleitet, und die Zysten werden ausgezaehlt.

Kartoffelmuedigkeit wird verursacht von Organismen oder Aelchen, die sich in die Stengel der Kartoffelpflanze einnisten und das Wachsen der Pflanze behindern oder ein Absterben der Pflanze bewirken.

Im Boden sind diese Aelchen in Zysten zu finden, die jede etwa 200 Aelchen enthalten und welche Aelchen sich in der Kartoffelpflanze vermehren koennen.

Die Zysten haben etwa die Groesse eines Sandkornes, waehrend die Aelchen in den Zysten, falls keine Kartoffelpflanze in der Naehe sind, um sich darin ein zu nisten, mehrere Jahre widerstandsfaehig sind gegen alles was in der Natur vorkommt.

Zur Bekaempfung der Organismen oder Aelchen, die Kartoffelmuedigkeit verursachen, wird denn auch Wechselbau angewendet, wobei dann unterschiedliche landwirtschaftliche Gewaechse in Aufeinanderfolge auf einem Acker angebaut werden um die Konzentration bestimmter Zysten im Boden einzuschraenken.

Zur Feststellung der Konzentration der Zysten im Boden wird wohl ein Verfahren angewendet, das bekannt ist als das Venwick-Verfahren, und wobei durch Spuelen in einer Venwickkanne die Zysten aus einer Bodenprobe losgelöst werden, sodass diese Zysten in der Kanne aufwaerts schwimmen um darauf mit dem uebrigen, leichteren Rueckstand abgesiebt zu werden.

Dieser Rueckstand mit den Zysten wird darauf nochmals mit Aceton behandelt, wodurch nur die Zysten aufwaerts schwimmen ; diese werden wiederum abgesiebt um unter einem Mikroskop gezaehlt zu werden.

Dieses bekannte Verfahren zur Feststellung der Konzentration der Zysten im Boden ist sehr umstaendlich, zeitraubend und ungenau. Ein anderes Auftriebsverfahren bzw. eine dafuer geeignete Vorrichtung ist in DD-A-44 999 beschrieben. Bei diesem bekannten Verfahren wird die Bodenprobe in einen Kolben hineingeschuettet und durch ein Strahlrohr mittels Wasserdruck vorgeweicht und durchgewirbelt, damit die vorhandenen Zysten ueber den Rand des Kolbens gespuelt werden um auf Sieben aufgefangen und gezaehlt zu werden. Auch dieses Verfahren ist sehr umstaendlich und ungenau.

Die Erfindung schafft ein neues Verfahren und eine Vorrichtung mittels denen die Konzentration der Zysten im Boden in einer schnellen und sehr genauen Weise festgestellt werden kann.

Das Verfahren gemaess der Erfindung ist in Anspruch 1 dargestellt.

Bei einer guenstigen Ausfuehrungsform des erfindungsgemaessen Verfahrens wird die mit Wasser vermischte Bodenprobe in einem zylindrischen Gefaess herumgeruehrt, wobei die an der Umgebungsluft grenzende und die Zysten enthaeltende oberste Schicht des Gemisches abgefiltert wird.

Beim Ruehren in dem zylindrischen Gefaess entsteht zentral in diesem Gefaess ein Strudeltrichter in dessen an die Aussenluft grenzende Aussenschicht sich die Zysten sammeln um am Ende des Ruehrens durch ein Sieb hindurch abgefiltert zu werden.

Die Vorrichtung zur Anwendung des erfindungsgemaessen Verfahrens ist in Anspruch 2 dargestellt.

Spezielle Ausgestaltungen der Vorrichtung sind in den abhaengigen Anspruechen dargestellt.

Beim Ruehren sammeln sich die Zysten mehr zentral im Gemischgefaess um darauf durch die Ueberlaufroehre abgefuehrt und durch die Abfiltersiebe abgefiltert zu werden.

Bei einer Ausfuehrungsform der Vorrichtung nach der Erfindung ist die Ueberlaufroehre in vertikaler Richtung verstellbar, und zwar derart, dass die oberste Schicht des Gemisches im Gefaess mit dem Verstellen der Ueberlaufroehre selektiv abgefuehrt, « abgerahmt » oder « abgeschaeumt » werden kann um mittels eines Siebes abgefiltert zu werden.

Auch ist es moeglich um waehrend des Ruehrens die offene Oberseite der Ueberlaufroehre immer auf einer gleichen Hoehe zu halten und die Wasserzufuhr in das die Bodenprobe enthaltende Mischgefaess derart zu regeln, dass die Fluessigkeitsoberflaeche im Ruehrtrichter zentral im Mischgefaess etwa gleich ist mit dem Ueberlaufrand der Ueberlaufroehre, sodass nach der Beendigung des Ruehrens und das sich beim Ausgleichen der Fluessigkeitsoberflaeche ueber den ganzen Querschnitt des Mischgefaesses, gerade die die Zysten enthaltende obere Schicht des Gemisches ueber den Ueberlaufrand der Ueberlaufroehre fliesst um darauf durch die Siebe hindurch abgefiltert zu werden.

Nach einem weiteren Merkmal der Vorrichtung gemaess der Erfindung gehoeren die Ruehrorgane zu einem antreibaren Ruehrwerk, das senkrecht in und aus dem Mischgefaess bewegbar ist.

Fuer das Hineinbringen der zu pruefenden Bodenprobe in das Mischgefaess kann dabei in guenstiger Weise das Ruehrwerk aus dem Mischgefaess hochgebracht werden um daraufhin, unter gleichzeitigem Einbringen von Wasser in das Mischgefaess, wieder nach unten in das Gefaess zurueck bewegt zu werden um das sich im Mischgefaess bildende Gemisch zu ruehren.

Das Auf- und Abbewegen des Ruehrwerkes kann auf unterschiedliche Weise bewirkt werden.

Bei einer sehr guenstigen Ausfuehrungsform der Vorrichtung gemaess der Erfindung ist das Ruehrwerk in einer senkrechten Richtung auf- und abbeweglich mittels einer vorzugsweise durch Wasserdruck betaetigbaren Kolben-Zylinder-Kombination.

Bei einer zweckmaesigen Ausfuehrungsform der Vorrichtung gemaess der Erfindung ist dazu das Mischgefaess im Boden versehen mit einer zentralen Abfuhr, die anschliesst an auf eine Abfuehrroehre und zusammenarbeitet mit dem unteren Ende der Ueberlaufroehre sodass mit dem Aufwaerts bewegen der Ueberlaufroehre die Abfuhr freigemacht wird und das Gemisch aus dem Gefaess wegstroemen kann.

Ebenso wie das Ruehrwerk kann auch die Ueberlaufroehre in zweckmaessiger Weise auf- und abbewegbar sein mittels einer vorzugsweise durch Wasserdruck betaetigbaren Kolben-Zylinder-Kombination.

Das Steuern der Kolben-Zylinder-Kombination des Ruehrwerkes und/oder der Ueberlaufroehre und die Regelung der Wasserzufuhr in das Mischgefaess kann in unterschiedlicher Weise stattfinden.

So kann die Wasserzufuhr in das Mischgefaess und/oder die Kolben-Zylinder-Kombination des Ueberlaufroehre und/oder des Ruehrwerks steuerbar sein mittels in der aufgenommener Zuleitung angebrachter elektro-magnetischer Absperrorgane.

Diese elektro-magnetischen Absperrorgane koennen wieder steuerbar sein mittels einer Programmschaltung, insbesondere mittels einer Programmschaltwalze.

Weitere Vorteile des Verfahrens und der Vorrichtung gemaess der Erfindung werden sich zeigen aus nachfolgender Beschreibung eines Ausfuehrungsbeispiels.

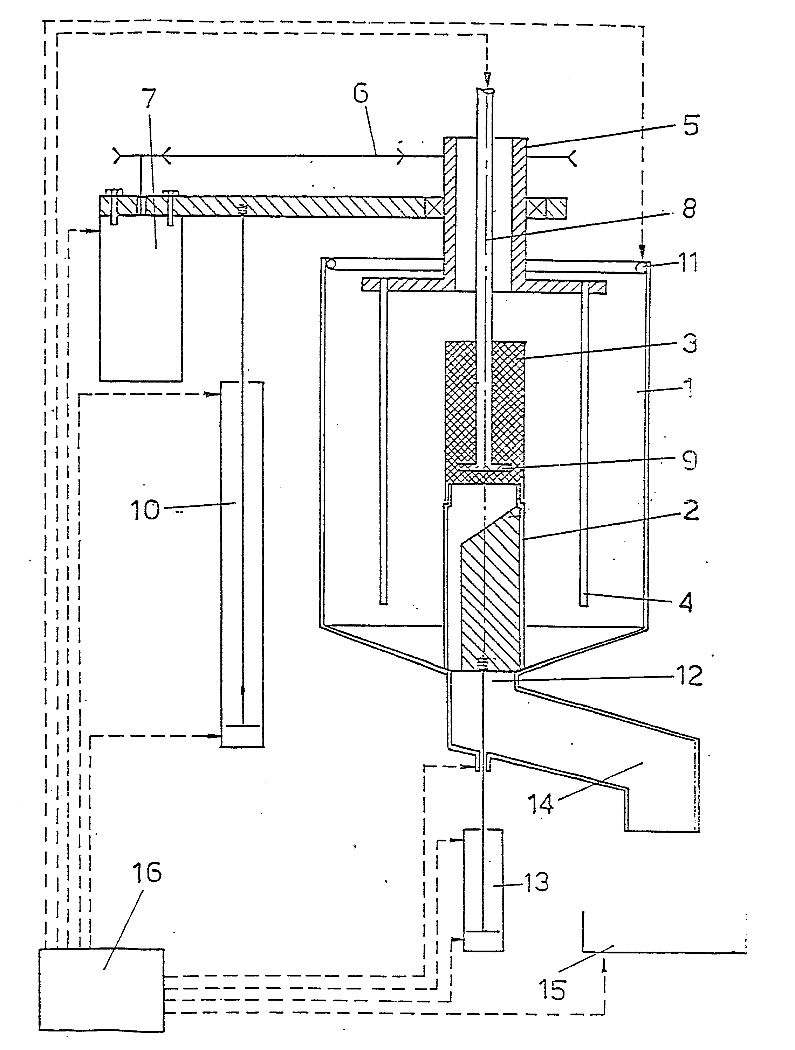

Wie in der Zeichnung dargestellt, wird die Vorrichtung gebildet von einem Mischgefaess 1, aus transparantem Kunststoff, in das zentral die Ueberlaufroehre 2 angebracht ist, und die an der offenen Oberseite mit dem Sieb 3 versehen ist.

Um die Ueberlaufroehre herum sind die Ruehrorgane 4 drehbar und gehoeren zu dem Ruehrwerk 5, das mittels des Seiles 6 von einem Elektromotor 7 angetrieben werden kann.

Am Ruehrwerk 5 ist ferner die Spritzroehre 8 mit der Spritzduese 9 angebracht.

Das Ruehrwerk 5 und damit auch die Spritzroehre 8 mit der Spritzduese 9 koennen ferner mittels der mit Wasserdruck betaetigbaren Kolben-Zylinder-Kombination 10 hinsichtlich des Mischgefaesses 1 auf- und abbewegt werden.

Mit dem Auf- und Abbewegen der Spritzroehre 8 kann die Ueberlaufroehre 2 mit dem darauf angebrachten Sieb 3 sauber gespritzt werden.

Im oberen Stand des Ruehrwerkes 5 sind die Ruehrorgane 4 aus dem Mischgefaess gehoben, sodass dieses mit der zu pruefenden Bodenprobe gefuellt werden kann.

Entlang dem oberen Rand des Mischgefaess 1 ist die Ringleitung 11 angeordnet, mittels deren Wasser ins Gefaess eingefuehrt werden kann. Das Mischgefaess 1 ist zentral an der Unterseite versehen mit einem Ablauf 12, welcher vom unteren Rand des Ueberlaufroehre 2 abgeschlossen ist, wenn diese sich im unteren Stand befindet.

Aus dieser unteren Lage kann die Ueberlaufroehre 2 mittels der vom Wasserdruck gesteuerten Kolben-Zylinder-Kombination 13 hochgebracht werden, wobei die Abfuhr 12 freikommt und die Fuellung des Mischgefaesses 1 durch die Leitung 14 hindurch wegstroemen kann nach dem Aufstellplatz 15 der Abfiltersiebe.

In die Wasserzufuhren der Ringleitung 11, die Spritzleitung 8 und die Kolben-Zylinder-Kombination 10 und 13 sind elektro-magnetische Absperrventile aufgenommen, welche von einer Programmschaltung 16 gesteuert werden koennen. Ebenfalls steuerbar von der Programmschaltung 16 aus ist der Elektromotor 7, waehrend die Programmschaltung 16 selbst in Betrieb gesetzt werden kann durch einen Schalter, der durch das Anbringen der Abfiltersiebe auf die Unterstuetzung 15 betaetigt ist. Der Schalter ist in der Zeichnung nicht dargestellt.

Zur Pruefung einer Bodenprobe wird diese in das Mischgefaess gebracht, mit Wasser vermischt, und das Gemisch wird einige Zeit geruehrt bei einer derartigen Hinzufuegung von Wasser, dass waehrend des Ruehrens das Niveau des Gemisches bis an den oberen Rand der in die untere Lage gebrachten Ueberlaufroehre kommt. Dabei steht das Gemisch durch die Ruehrwirkung an der Wand im Mischgefaess hoeher als im mehr zentral gebildeten Strudeltrichter.

Nach dem Ruehren kommt die Fluessigkeitsoberflaeche im Mischgefaess wieder in der Horizontalebene zu liegen, wobei die waehrend des Ruehrens in der obersten Schicht des Gemisches gesammelten Zysten mit dieser Schicht ueber den Rand der Ueberlaufroehre 2 in diese und darauf durch die Leitung 14 hindurch abgefuehrt werden auf die Unterstuetzung 15 angebrachten Abfiltersiebe, auf der die Zysten zurueckbleiben um gesaehlt zu werden zur Festellung der Zystenkonzentration in der Grundprobe. Nachdem das Abfiltersieb mit den darauf abgefilterten Zysten weggenommen ist, wird die Ueberlaufroehre 2 in die obere Lage bewegt, wodurch der Ablauf 12 freigegeben wird und das Mischgefaess 1 durch die Leitung 14 leerlaufen kann.

Je nach dem Programm der Programmschaltung 16 kann die Spritzroehre 8 mit der Duese 9 auf- und abbewegt werden durch die Kolben-Zylinder-Kombination 10 zum Reinigen des Inneren der Ueberlaufroehre 2 und des Siebes 3, worauf die Vorrichtung wieder bereit ist fuer die Aufnahme einer neuen Bodenprobe, mit deren Pruefung angefangen wird mit dem Anbringen der Abfiltersiebe auf die Unterstuetzung 15.

**Patentansprüche**

1. Verfahren fuer das Absondern der Zysten von Kartoffelmuedigkeit verursachenden Organismen oder Aelchen aus eine Bodenprobe, wobei die Bodenprobe mit Wasser vermischt wird und die Zysten sich als leichtere Bestandteile an der Gemischoberflaeche sammeln um mit einem Teil des Wassers in einem Abfiltersieb aufgefangen und zur Feststellung der Konzentration gezaehlt zu werden, dadurch gekennzeichnet, dass die mit Wasser vermischte Bodenprobe einer Zentrifugalkraft unterworfen wird, worauf die an die Umgebungsluft grenzende und die Zysten enthaltende oberste Schicht des Gemisches abgefiltert wird.

2. Vorrichtung zur Anwendung des Verfahrens nach Anspruch 1, gebildet durch ein Mischgefaess (1) zur Aufnahme der zu pruefenden Bodenprobe und des damit zu vermischenden Wassers, dadurch gekennzeichnet, dass zentral im Mischgefaess (1) eine Ueberlaufroehre (2) und darum herum ein oder mehrere antreibbare Ruehrorgane (4) fuer das In-Drehung-Versetzen der mit dem Wasser vermischten Bodenprobe angebracht sind.

3. Vorrichtung nach Anspruch 2, dadurch gekennzeichnet, dass die Ueberlaufroehre (2) in vertikaler Richtung verstellbar ist, sodass die oberste Schicht des Gemisches im Gefaess (1) mit dem Verstellen der Ueberlaufroehre (2) selektiv abgefuehrt und von einem Sieb abgefiltert werden kann.

4. Vorrichtung nach Anspruch 2 oder 3, dadurch gekennzeichnet, dass die Ueberlaufroehre (2) am offenen oberen Ende mit einem Sieb (3) zur Rueckhaltung der groeberen Rueckstandsteile des Gemisches versehen ist.

5. Vorrichtung nach Anspruch 2, 3 oder 4, dadurch gekennzeichnet, dass das Mischgefaess (1) entlang dem oberen Umrissrand mit einer Ringleitung (11) fuer das Einfuehren von Wasser ins Gefaess versehen ist.

6. Vorrichtung nach Anspruch 2, dadurch gekennzeichnet, dass die Ruehrorgane (4) zu einem antreibbaren Ruehrwerk (5), das senkrecht in und aus dem Mischgefaess (1) bewegbar ist, gehoeren.

7. Vorrichtung nach Anspruch 6, dadurch gekennzeichnet, dass das Ruehrwerk (5) in senkrechter Richtung mittels einer vorzugsweise durch Wasserdruck steuerbaren, Kolben-Zylinder-Kombination auf- und abbwegbar ist.

8. Vorrichtung nach einem der vorangehenden Ansprueche, dadurch gekennzeichnet, dass die Ueberlaufroehre (2) an eine Ablaufleitung (14) anschliesst, welche oberhalb einer am Gestell oder am Gehaeuse der Vorrichtung angebrachten Unterstuetzung (15) zur Aufnahme eines Abfiltersiebes, mit welchem die Zysten abgefiltert werden, muendet.

9. Vorrichtung nach einem der vorangehenden Ansprueche, dadurch gekennzeichnet, dass an das Ruehrwerk (5) und zwischen den Ruehrorganen (4) eine in die Ueberlaufroehre (2) hineinbringbare Wasserspritzduese (9) angebracht ist, mittels derer die Ueberlaufroehre (2) und gegebenenfalls das darauf angebrachte Sieb (3) gereinigt werden koennen.

10. Vorrichtung nach einem der vorangehenden Ansprueche, dadurch gekennzeichnet, dass das Mischgefaess (1) im Boden mit einem zentralen Ablauf (12) versehen ist und dass beim Aufwaertsbewegen der Ueberlaufroehre (2) der Ablauf (12) freigemacht wird und das Gemisch aus dem Gefaess (1) abstroemen kann.

11. Vorrichtung nach einem der vorangehenden Ansprueche, dadurch gekennzeichnet, dass die Ueberlaufroehre (2) mittels einer vorzugsweise durch Wasserdruck steuerbaren Kolben-Zylinder-Kombination (13) auf- und abbewegbar ist.

12. Vorrichtung nach einem der vorangehenden Ansprueche, dadurch gekennzeichnet, dass die Wasserzufuhr in das Mischgefaess (1) und/oder die Kolben-Zylinder-Kombinationen (10, 13) der Ueberlaufroehre (2) bzw. des Ruehrwerks mittels eines in die Zufuhr aufgenommenen elektromagnetischen Absperrorgans steuerbar sind.

13. Vorrichtung nach Anspruch 12, dadurch gekennzeichnet, dass das elektro-magnetische Absperrorgan oder die elektromagnetischen Absperrorgane mittels einer Programmschaltung, insbesondere mittels einer Programmschaltwalze, steuerbar ist bzw. sind.

14. Vorrichtung nach einem der vorangehenden Ansprueche, dadurch gekennzeichnet, dass in der Naehe der Unterstuetzung (15) zur Aufnahme der Abfiltersiebe ein Schalter angebracht ist, welcher beim Aufstellen der Abfiltersiebe die Programmschaltung in Betrieb setzt.

**Claims**

1. Process for isolating the cysts of organisms or eelworms causing potato-disease from a soil sample, in which the soils sample is mixed with water and the cysts collect as lighter components at the surface of the mixture to be catched with a portion of the water in a filter-sieve and to be counted to determine the concentration, characterized in that the soil sample mixed with water is subjected to a centrifugal force, after which the upper layer of the mixture, which borders the surrounding air and contains the cysts, is filtered off.

2. Device for applying the process as claimed in claim 1, formed by a mixing vessel (1) for receiving the soil sample to be examined and the water to be mixed therewith, characterized in that centrally in the mixing vessel (1) an overflowpipe (2) and around this overflowpipe one or more stirring members (4) have been mounted to bring the soil sample mixed with water into rotation.

3. Device as claimed in claim 2, characterized in that overflowpipe (2) is adjustable in a vertical direction, so that the upper layer of the mixture in the vessel (1) by adjusting the overflowpipe (2) selectively may be drained off and filtered off by a sieve.

4. Device as claimed in claim 2 or 3, characterized in that the overflowpipe (2) at its open upper end is provided with a sieve (3) to retain the coarser components of the remainders of the mixture.

5. Device as claimed in claim 2, 3 or 4, characterized in that the mixing vessel (1) along its upper circumferential rim is provided with an annual conduit (11) for the supply of water into the vessel.

6. Device as claimed in claim 2, characterized in that the stirring members (4) form part of a stirring apparatus (5) which may be moved into and out of the mixing vessel (1).

7. Device as claimed in claim 6, characterized in that the stirring apparatus (5) may be moved up and down in vertical direction by means of a piston-cilinder-combination which preferably may be controlled by water-pressure.

8. Device as claimed in one of the preceding claims, characterized in that the overflowpipe (2) is connected to a drain conduit (14) debouching above a support (15) mounted onto the frame or the housing of the device, and which support is intended to receive a filtering sieve, by means of which the cysts may be filtered off.

9. Device as claimed in one of the preceding claims, characterized in that onto the stirring apparatus (5) and between the stirring members (4) a water spray nozzle (9) is mounted, which may be brought into the overflowpipe (2) and by means of which the overflowpipe (2) and eventually the sieve (3) mounted theron may be cleaned.

10. Device as claimed in one of the preceding claims, characterized in that the mixing vessel (1) is provided in its bottom with a central drain (12) and that by moving the overflowpipe (2) upwards, the drain is set free for draining off the mixture from the vessel (1).

11. Device as claimed in one of the preceding claims, characterized in that the overflowpipe (2) may be moved up and down by means of a piston-cilinder-combination (13) which preferably may be controlled by water-pressure.

12. Device as claimed in one of the preceding claims, characterized in that the water supply into the mixing vessel (1) and/or the piston-cilinder-combinations (10, 13) of the overflowpipe (2) resp. the stirring apparatus may be controlled by an electro-magnetic valve mounted in the supplying conduit.

13. Device as claimed in claim 12, characterized in that the electro-magnetic valve or the electro-magnetic-valves may be controlled by means of a program-circuit, particularly by a program-drum-switch.

14. Device as claimed in one of the preceding claims, characterized in that near the support (15) for the filter-sieve a switch is mounted which sets the programcircuit into operation when the sieve is installed onto the support.

## Revendications

1. Procédé pour traiter un échantillon prélevé dans le sol, en vue d'en séparer les kystes dus aux organismes qui provoquent une maladie des pommes de terre ou les anguillules correspondantes ; ce procédé consistant à diluer l'échantillon de sol dans de l'eau pour rassembler à la surface du mélange les kystes qui sont relativement plus légers, afin de les retenir avec une partie de l'eau dans un tamis de filtrage, en vue d'un comptage pour en déterminer la concentration, procédé caractérisé en ce qu'on centrifuge l'échantillon de sol mélangé avec de l'eau, et en ce qu'on sépare ensuite par filtrage la couche supérieure du mélange qui est au contact de l'air ambiant et qui contient les kystes.

2. Dispositif pour la mise en œuvre du procédé conforme à la revendication 1, comportant un récipient de mélange (1) destiné à recevoir l'échantillon de sol à traiter et l'eau à mélanger à cet échantillon, dispositif caractérisé en ce qu'il comporte un tube de trop-plein (2) disposé au centre du récipient de mélange (1), et au moins un organe agitateur (4) qui est aménagé autour dudit tube et qui est associé à un système d'entraînement pour mettre en rotation l'échantillon de sol mélangé avec de l'eau.

3. Dispositif selon la revendication 2, caractérisé en ce que le tube de trop-plein (2) est réglable dans le sens vertical, pour permettre l'évacuation sélective de la couche supérieure du mélange contenu dans le récipient (1), grâce à un réglage approprié du tube de trop-plein (2), en vue d'une séparation par filtrage.

4. Dispositif selon l'une des revendications 2 ou 3, caractérisé en ce que le tube de trop-plein (2) est pourvu à l'endroit de son extrémité ouverte supérieure d'un tamis (3), pour retenir les plus grosses particules résiduelles du mélange.

5. Dispositif selon l'une des revendications 2 à 4, caractérisé en ce que le récipient de mélange (1) comporte le long de son bord supérieur une canalisation annulaire (11) pour introduire de l'eau dans ledit récipient.

6. Dispositif selon la revendication 2, caractérisé en ce que chacun des organes agitateurs (4) fait partie d'un mécanisme d'agitation (5) qui est associé à un système d'entraînement, et qui est mobile dans le sens vertical pour pouvoir être introduit dans le récipient de mélange (1) et retiré de ce récipient.

7. Dispositif selon la revendication 6, caractérisé en ce que le mécanisme d'agitation (5) peut être déplacé dans le sens vertical au moyen d'un vérin de manœuvre de préférence hydraulique, pour être mis en place et retiré.

8. Dispositif selon l'une quelconque des revendications précédentes, caractérisé en ce que le tube de trop-plein (2) est relié à une canalisation d'évacuation (14), qui débouche au-dessus d'un support (15) fixé au bâti ou au boîtier du dispositif et prévu pour recevoir un tamis de séparation qui sert à retenir les kystes.

9. Dispositif selon l'une quelconque des revendications précédentes, caractérisé en ce qu'il comporte une buse de projection d'eau (9) adaptée à être enfoncée dans le tube de trop-plein (2), pour nettoyer ledit tube de trop-plein (2) ainsi qu'éventuellement le tamis (3) monté sur ce tube.

10. Dispositif selon l'une quelconque des revendications précédentes, caractérisé en ce que le récipient de mélange (1) comporte au fond un orifice d'évacuation (12) adapté de manière à être libéré par un soulèvement du tube de trop-plein (2), pour permettre l'écoulement du mélange hors du récipient (1).

11. Dispositif selon l'une quelconque des revendications précédentes, caractérisé en ce que le tube de trop-plein (2) est associé à un vérin de manœuvre (13), de préférence hydraulique, qui permet de soulever et d'abaisser ledit tube de trop-plein.

12. Dispositif selon l'une quelconque des revendications précédentes, caractérisé en ce qu'il comporte une électro-vanne d'arrêt montée sur l'arrivée d'eau pour commander l'alimentation en eau du récipient de mélange (1) et/ou celle des vérins de manœuvre (10, 13) associés au mécanisme d'agitation et au tube de trop-plein (2).

13. Dispositif selon la revendication 12, caractérisé en ce que chaque électro-vanne est commandée par un système de commutateurs à programme, tel qu'un système à tambour.

14. Dispositif selon l'une quelconque des revendications précédentes, caractérisé en ce qu'il comporte un contacteur disposé au voisinage d'un support (15) prévu pour recevoir des tamis de séparation, afin de commander la mise en marche d'un système de commutateurs à programme lors de la mise en place desdits tamis de séparation.